(19) Europäisches Patentamt
European Patent Office
Office européen des brevets

(11) Veröffentlichungsnummer : **0 455 657 B1**

(12) # EUROPÄISCHE PATENTSCHRIFT

(45) Veröffentlichungstag der Patentschrift : **14.07.93 Patentblatt 93/28**

(51) Int. Cl.$^5$ : **A61K 7/075,** C11D 1/37

(21) Anmeldenummer : **90901802.0**

(22) Anmeldetag : **17.01.90**

(86) Internationale Anmeldenummer : **PCT/EP90/00092**

(87) Internationale Veröffentlichungsnummer : **WO 90/08531 09.08.90 Gazette 90/19**

(54) **OBERFLÄCHENAKTIVE MISCHUNGEN.**

(30) Priorität : **25.01.89 DE 3902048**

(43) Veröffentlichungstag der Anmeldung : **13.11.91 Patentblatt 91/46**

(45) Bekanntmachung des Hinweises auf die Patenterteilung : **14.07.93 Patentblatt 93/28**

(84) Benannte Vertragsstaaten : **AT BE CH DE ES FR GB IT LI LU NL SE**

(56) Entgegenhaltungen :
**EP-A- 0 349 906**
**DE-A- 3 725 030**

(73) Patentinhaber : **Henkel Kommanditgesellschaft auf Aktien
Postfach 1100 Henkelstrasse 67
W-4000 Düsseldorf-Holthausen (DE)**

(72) Erfinder : **MÜLLER, Reinhard
Gottfried Hausmann Weg 13
W-5140 Erkelenz 7 (DE)**
Erfinder : **FABRY, Bernd
Danziger Strasse 31
W-4052 Korschenbroich (DE)**
Erfinder : **THUL, Jutta
Händelstrasse 26
W-4010 Hilden (DE)**
Erfinder : **WISOTZKI, Klaus-Dieter
Neuendickstrasse 9
W-4132 Kamp-Lintfort (DE)**
Erfinder : **GIEDE, Karl
Schlehenweg 12
W-4010 Hilden (DE)**

**EP 0 455 657 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft oberflächenaktive Mischungen mit geringer Hautbelastung.

Um die Reinigungswirkung wäßriger Zubereitungen zu verstärken, enthalten diese üblicherweise oberflächenaktive Verbindungen. Allerdings ist eine gesteigerte Reinigungswirkung in der Regel mit einer erhöhten Hautbelastung verbunden. Dies gilt besonders für die wichtige Klasse der Aniontenside.

Diese erhöhte Hautbelastung sollte im Bereich der Körperreinigungsmittel vermieden werden. Insbesondere bei Produkten, die für eine häufige Anwendung konzipiert sind, zur Reinigung des Intimbereiches dienen oder mit Schleimhäuten in Berührung kommen, ist eine gute Hautverträglichkeit wichtig.

Es besteht daher ein Bedarf an Körperreinigungsmitteln, die sich bei ausreichender Reinigungsleistung und zufriedenstellenden anderen Eigenschaften durch eine deutlich geringere Hautbelastung auszeichnen.

Es ist bekannt, daß durch Einsatz von Magnesiumionen bei Aniontensiden wie den Fettalkoholsulfaten und Fettalkoholethersulfaten Produkte mit verbesserter Hautverträglichkeit erhalten werden können.

Gleichfalls bekannt ist, daß sich ampholytische und nichtionogene Tenside im Vergleich zu Aniontensiden häufig durch eine verbesserte Hautverträglichkeit auszeichnen. Diese Tensidklassen können jedoch für viele Anwendungsbereiche nicht als Ersatz für Aniontenside verwendet werden, da sie in der Reinigungswirkung deren Eigenschaften nicht erreichen. Auch die für Shampoos gewünschte Schaumbildung ist bei ausschließlicher oder überwiegender Verwendung von Amphotensiden in der Regel nicht gewährleistet.

Aus einem in "Ärztliche Kosmetologie 13, 39-45 (1983)" veröffentlichten Artikel ist zu entnehmen, daß die Hautverträglichkeit von Anion-/Amphotensid-Mischungen in Einzelfällen über der der reinen Aniontenside liegt. Somit läßt sich die Hautverträglichkeit von Tensidlösungen dadurch steigern, daß die Aniontenside teilweise durch Amphotenside ersetzt werden, es müssen jedoch hinsichtlich wichtiger Eigenschaften wie Reinigungswirkung und Schaumbildung Nachteile in Kauf genommen werden.

Es bestand daher die Aufgabe, Reinigungsmittel zu finden, die sich sowohl durch eine hohe Reinigungsleistung und Schaumbildung, als auch durch eine deutlich geringere Hautbelastung auszeichnen.

Es wurde nun gefunden, daß bei bestimmten Kombinationen von Aniontensiden eine synergistische Zunahme der Hautverträglichkeit auftritt. Auf Basis dieser Tensidkombinationen ist die Formulierung von Reinigungsmitteln möglich, die die Vorteile von Aniontensiden wie hohe Reinigungsleistung und große Schaumbildung mit einer gesteigerten Hautverträglichkeit verbinden.

Gegenstand der Erfindung sind oberflächenaktive wäßrige Mischungen enthaltend mindestens zwei unterschiedliche Aniontenside, dadurch gekennzeichnet, daß als Aniontenside

a) ein Gemisch oberflächenaktiver innenständiger Ethersulfonate (A), das ganz oder überwiegend aus Verbindungen der Formeln (I) und/oder (II)

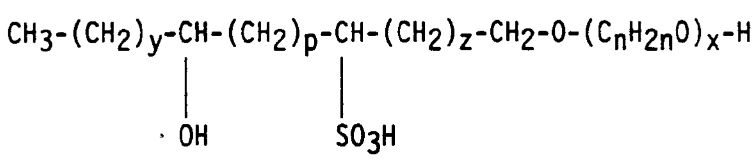

(I)

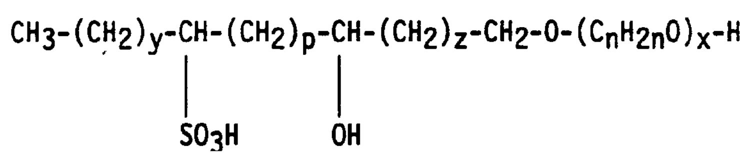

(II)

oder deren Alkali-, Erdalkali-, Aluminium- und Ammoniumsalzen zusammengesetzt ist, wobei y und z = 0 oder Zahlen von 1 bis 18, p = 0, 1 oder 2 und die Summe (y+z+p) eine Zahl von 12 bis 18, x = 0 oder eine ganze Zahl bis 30 und n eine ganze Zahl von 2 bis 4 sein kann,

und

b) mindestens eine weitere Verbindung (B), ausgewählt aus der Gruppe der Fettalkoholethersulfate, Fettalkoholsulfate, Ethercarbonsäuren, Sulfobernsteinsäureester, Acylsarkoside, Acyltauride, Acylisethionate, α-Olefinsulfonate und sekundären Alkylsulfonate, enthalten sind.

Die oberflächenaktiven innenständigen Ethersulfonate (A) sind Gegenstand der Deutschen Patentameldung P 37 25 030.2 . Die Verbindungen sind durch Umsetzung der ungesättigten Fettalkyl- oder Fettalkylpolyoxyalkyl-ester mit $SO_3$ und anschließende Aufarbeitung des Umsetzungproduktes mit wäßrigem Alkali-, Erdalkali- oder Ammoniumhydroxid zugänglich. Bezüglich der Einzelheiten des Herstellungsverfahrens wird ausdrücklich auf den Inhalt der genannten Patentanmeldung P 37 25 030.2 Bezug genommen.

Bei diesem Herstellungsverfahren können in Abhängigkeit von den gewählten Reaktionsbedingungen Nebenprodukte auftreten, die durch Wasserabspaltung aus Verbindungen gemäß Formel (I) oder Formel (II) entstehen. Diese Nebenprodukte, die durch den Austritt der OH-Gruppe sowie eines Wasserstoffatoms an einem der beiden Kohlenstoffatome gebildet werden, die dem die OH-Gruppe tragenden Kohlenstoffatom benachbart sind, können in ungünstigen Fällen bis zu 50 Gew.-% der gesamten Produktmenge darstellen. Auch solche Mischungen von Verbindungen der Formeln (I) und/oder (II) und den genannten Nebenprodukten sind innenständige Ethersulfonate (A) im Rahmen der erfindungsgemäßen Lehre.

Die innenständigen Ethersulfonate (A) sind in der oberflächenaktiven wäßrigen Mischung in Mengen von 1 bis 99 Gew.%, bezogen auf die Gesamtmenge der Aniontenside, enthalten.

Bevorzugte innenständige Ethersulfonate (A) sind die ethoxylierten Verbindungen, bei denen x eine Zahl von 3 bis 15, insbesondere von 5 bis 10 darstellt.

Verbindungen gemäß Formel (I) oder (II), bei denen (y+z+p) = 14 und x = 10 ist, sind ganz besonders bevorzugte Komponenten A. Darunter fallen beispielsweise die aus dem Umsetzungsprodukt von Oleylalkohol mit etwa 10 Mol Ethylenoxid zugänglichen innenständigen Ethersulfonate.

Es ist dem Fachmann bekannt, daß bei Alkoxylierungsreaktionen wie beispielsweise der Anlagerung von x mol Ethylenoxid an 1 mol Fettalkohol nach den bekannten Ethoxylierungsverfahren kein einheitliches Addukt, sondern ein Gemisch aus Restmengen freien Fettalkohols und einer Reihe homologer (oligomerer) Anlagerungsprodukte von 1, 2, 3, ... x, x+1, x+2 ...usw. Molekülen Ethylenoxid je Molekül Fettalkohol erhalten wird. Der mittlere Ethoxylierungsgrad (x) wird dabei definiert durch die Ausgangsmengen an Fettalkohol und Ethylenoxid. Die Verteilungskurve des Homologengemisches weist in der Regel ein Maximum im Bereich zwischen x-3 und x+3 auf. Nähere Informationen hierzu können beispielsweise der Zeitschrift Soap/Cosmetics/Chemical Specialities, Heft Januar 1988, S. 34, entnommen werden.

Unter Fettalkoholethersulfaten sind die Sulfate der Anlagerungsprodukte von Ethylenoxid (EO) und/oder Propylenoxid (PO) an gesättigte und/oder ungesättigte, lineare und/oder verzweigte Fettalkohole zu verstehen, die nach bekannten Verfahren zugänglich sind. Die den Fettalkoholethersulfaten zugrunde liegenden Fettalkohole können reine Verbindungen sein. Es ist jedoch üblicherweise bevorzugt, Mischungen aus verschiedenen Fettalkoholen zu verwenden, die aus nativen Rohstoffen wie Fetten und Ölen erhalten werden. Diese Fettalkohole können mit den Alkylenoxiden beispielsweise unter Druck und bei Anwesenheit von Katalysatoren zu Fettalkoholalkoxylaten umgesetzt werden. Bezüglich des Alkoxylierungsgrades gilt hier in gleicher Weise das bei der Beschreibung der Hydroxysulfonate Gesagte. Durch Umsetzung der Fettalkoholalkoxylate mit beispielsweise Schwefeltrioxid oder Chlorsulfonsäure und anschließender Neutralisation, beispielsweise mit Alkalimetall-, Erdalkalimetall-, Aluminium- oder Ammoniumhydroxiden, werden schließlich die gewünschten Fettalkoholethersulfate erhalten.

Bevorzugte Fettalkoholethersulfate (B1) sind Verbindungen gemäß Formel (III),

$$R\text{-}O\text{-}(C_mH_{2m}O)_v\text{-}SO_3X \qquad (III)$$

in der R ein gesättigter oder olefinisch ungesättigter, linearer oder verzweigter Alkylrest mit 8 bis 22 C-Atomen, m 2 oder 3, v eine Zahl von 1 bis 15 und X Wasserstoff, ein Alkalimetall, ein Erdalkalimetall, Aluminium, eine Ammoniumgruppe oder eine Alkyl- oder Alkylolammoniumgruppe mit 1 bis 4 C-Atomen in jeder Alkyl- oder Alkylolgruppe ist.

Besonders bevorzugt sind die Verbindungen, bei denen R ein gesättigter Alkylrest mit 8 bis 16 C-Atomen, m = 2, v eine Zahl von etwa 2 bis 10 und X Natrium, Kalium, Magnesium, Aluminium, Ammonium oder eine Mono-, Di- oder Triethanolammoniumgruppe ist.

Besonders vorteilhafte Synergien bezüglich der Hautverträglichkeit wurden bei Mischungen gefunden, die die innenständigen Ethersulfonate (A) und die Fettalkoholethersulfate (B1) in einem Mengenverhältnis von etwa 10:90 bis etwa 70:30, insbesondere von 20:80 bis 60:40, enthalten.

Bevorzugte Fettalkoholsulfate (B2) sind Verbindungen gemäß Formel (IV),

$$R\text{-}O\text{-}SO_3X \qquad (IV)$$

in der R ein gesättigter oder olefinisch ungesättigter, linearer oder verzweigter Alkylrest mit 8 bis 22 C-Atomen und X Wasserstoff, ein Alkalimetall, ein Erdalkalimetall, Aluminium, eine Ammoniumgruppe oder eine Alkyl- oder Alkylolammoniumgruppe mit 1 bis 4 C-Atomen in jeder Alkyl- oder Alkylolgruppe ist.

Besonders bevorzugt sind die Verbindungen, bei denen R ein gesättigter Alkylrest mit 8 bis 16 C-Atomen und X Natrium, Kalium, Magnesium, Aluminium, Ammonium oder eine Mono-, Di- oder Tri- ethanolammoniumgruppe ist.

Besonders vorteilhafte Synergien bezüglich der Hautverträglichkeit wurden bei Mischungen gefunden, die die innenständigen Ethersulfonate (A) und die Fettalkoholsulfate (B2) in einem Mengenverhältnis von etwa 10:90 bis etwa 50:50, insbesondere von 10:90 bis 30:70, enthalten.

Bevorzugte Ethercarbonsäuren (B3) sind Verbindungen gemäß Formel (V),

$$R\text{-}O\text{-}(C_mH_{2m}O)_v\text{-}CH_2\text{-}COOX \qquad (V)$$

in der R ein gesättigter oder olefinisch ungesättigter, linearer oder verzweigter Alkylrest mit 8 bis 22 C-Atomen, m 2 oder 3, v eine Zahl von 1 bis 15 und X Wasserstoff, ein Alkalimetall, ein Erdalkalimetall, Aluminium, eine Ammoniumgruppe oder eine Alkyl- oder Alkylolammoniumgruppe mit 1 bis 4 C-Atomen in jeder Alkyl- oder Alkylolgruppe ist.

Besonders bevorzugt sind die Verbindungen, bei denen R ein gesättigter Alkylrest mit 8 bis 16 C-Atomen, m = 2, v eine Zahl von etwa 5 bis 12, insbesondere etwa 10, und X Natrium, Magnesium, Aluminium, Ammonium oder eine Mono-, Di- oder Triethanolammoniumgruppe ist.

Ethercarbonsäuren der genannten Art werden beispielsweise von der Firma CHEM-Y unter der Bezeichnung "Akypo(R)" vertrieben.

Besonders vorteilhafte Synergien bezüglich der Hautverträglichkeit wurden bei Mischungen gefunden, die die innenständigen Ethersulfonate (A) und die Ethercarbonsäuren (B3) in einem Mengenverhältnis von etwa 10:90 bis etwa 90:10, insbesondere von 20:80 bis 80:20, enthalten.

Bevorzugte Sulfobernsteinsäureester (B4) sind Verbindungen gemäß Formel (VI),

$$R\text{-}(OC_mH_{2m})_w\text{-}O\text{-}OC\text{-}CH_2\text{-}\underset{\displaystyle SO_3Z}{\underset{|}{CH}}\text{-}COOZ' \qquad (VI)$$

in der R ein gesättigter oder olefinisch ungesättigter, linearer oder verzweigter Alkylrest mit 8 bis 22 C-Atomen, m 2 oder 3, w = 0 oder eine Zahl von 1 bis 15 und Z ein Alkalimetall, ein Erdalkalimetall, Aluminium, eine Ammoniumgruppe, eine Alkyl- oder Alkylolammoniumgruppe mit 1 bis 4 C-Atomen in jeder Alkyl- oder Alkylolgruppe und Z' ein Alkalimetall, ein Erdalkalimetall, Aluminium, eine Ammoniumgruppe, eine Alkyl- oder Alkylolammoniumgruppe mit 1 bis 4 C-Atomen in jeder Alkyl- oder Alkylolgruppe oder eine Gruppe R ist.

Besonders bevorzugt sind die Sulfobernsteinsäurehalbester. Bei diesen Verbindungen ist Z' ein Alkalimetall, ein Erdalkalimetall, Aluminium, eine Ammoniumgruppe oder eine Alkyl- oder Alkylolammoniumgruppe mit 1 bis 4 C-Atomen in jeder Alkyl- oder Alkylolgruppe. Besonders gut geeignet sind die Verbindungen, bei denen R ein gesättigter Alkylrest mit 8 bis 16 C-Atomen, m = 2, w eine Zahl von etwa 5 bis 12 und Z Natrium ist.

Besonders vorteilhafte Synergien bezüglich der Hautverträglichkeit wurden bei Mischungen gefunden, die die innenständigen Ethersulfonate (A) und die Sulfobernsteinsäureester (B4) in einem Mengenverhältnis von etwa 10:90 bis etwa 90:10, insbesondere von 20:80 bis 80:20, enthalten.

Die als weitere Aniontensidklasse erfindungsgemäß einsetzbaren Acylsarkoside sind Umsetzungsprodukte von Fettsäuren mit N-Methylglycin mit der allgemeinen Formel $R\text{-}CO\text{-}N(CH_3)\text{-}CH_2\text{-}COOH$, wobei R-CO einen Fettacylrest mit 8-22 C-Atomen, bevorzugt mit 10-18 C-Atomen, darstellt. Bei den Acyltauriden handelt es sich um Umsetzungsprodukte von Fettsäurechloriden mit N-Methyltaurin mit der allgemeinen Formel $R\text{-}CO\text{-}N(CH_3)\text{-}CH_2\text{-}CH_2\text{-}SO_3Na$, wobei R-CO einen Fettacylrest mit 8-22 C-Atomen, insbesondere mit 10-18 C-Atomen, darstellt und an Stelle des Natriums auch andere Gegenionen wie beispielsweise andere Alkalimetallionen, Erdalkalimetallionen oder Ammoniumionen vorliegen können.

Acylisethionate werden üblicherweise durch Umsetzung von Fettsäurechloriden mit Natriumisethionat hergestellt und haben die allgemeine Formel $R\text{-}CO\text{-}O\text{-}CH_2\text{-}CH_2\text{-}SO_3Na$, wobei R-CO einen Fettacylrest mit 8-22 C-Atomen, insbesondere mit 10-18 C-Atomen, darstellt und an Stelle des Natriums auch andere Gegenionen wie beispielsweise andere Alkalimetallionen, Erdalkalimetallionen oder Ammoniumionen vorliegen können.

$\alpha$-Olefinsulfonate werden üblicherweise durch Sulfonierung von $\alpha$-Olefinen mit $SO_3$ und anschließende Hydrolyse der so erhaltenen Sultone hergestellt. Das als $\alpha$-Olefinsulfonat bezeichnete Produkt besteht in der Regel zu etwa 60 % aus Alkensulfonaten der Form $R^1\text{-}CH_2\text{-}CH{=}CH\text{-}(CH_2)_k\text{-}SO_3Na$ und zu etwa 40 % aus Hydroxyalkansulfonaten der Formel $R^2\text{-}CH_2\text{-}CHOH\text{-}(CH_2)_f\text{-}SO_3Na$, wobei $R^1$ eine Alkylgruppe mit etwa 8 bis 13 C-Atomen, $R^2$ eine Alkylgruppe mit etwa 7 bis 13 C-Atomen, und k und f unabhängig voneinander eine Zahl 1, 2 oder 3 ist.

Sekundäre Alkylsulfonate sind durch Sulfochlorierung oder Sulfoxidation linearer Paraffine zugänglich und haben die allgemeine Formel $R\text{-}CH(SO_3Na)\text{-}R'$, wobei R und R' lineare Alkylgruppen mit 1-10 C-Atomen darstellen, wobei die Summe der C-Atome in R und R' 10 bis 20 ist, und an Stelle des Natriums auch andere Gegenionen wie beispielsweise andere Alkalimetallionen, Erdalkalimetallionen oder Ammoniumionen vorliegen können.

Die erfindungsgemäßen oberflächenaktiven Mischungen enthalten insgesamt etwa 2-50 Gew.-% an Aniontensiden. Ein Gehalt von 5-30 Gew.-% ist bevorzugt.

Weiterhin können die erfindungsgemäßen oberflächenaktiven Mischungen neben den Aniontensiden zusätzlich 0,5-20 Gew.-%, insbesondere 1-10 Gew.-%, an ampholytischen und/oder zwitterionischen Tensiden enthalten. Unter ampholytischen Tensiden werden solche oberflächenaktiven Verbindungen verstanden, die außer einer $C_8$-$C_{18}$-Alkyl- oder -Acylgruppe im Molekül mindestens eine freie Aminogruppe und mindestens eine -COOH- oder -$SO_3$H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarkosine, 2-Alkylaminopropionsäuren und Alkylaminoessigsäuren mit jeweils etwa 8 bis 18 C-Atomen in der Alkylgruppe.

Als zwitterionische Tenside werden solche oberflächenaktiven Verbindungen bezeichnet, die im Molekül mindestens eine quartäre Ammoniumgruppe und mindestens eine -$COO^{(-)}$- oder -$SO_3^{(-)}$-Gruppe tragen. Besonders geeignete zwitterionische Tenside sind die sogenannten Betaine wie die N-Alkyl-N,N-dimethylammonium-glycinate, beispielsweise das Kokosalkyl-dimethylammonium-glycinat, N-Acylaminopropyl-N,N-dimethylammoniumglycinate, beispielsweise das Kokosacylaminopropyl-dimethylammoniumglycinat, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat.

Die hautschonenden Eigenschaften der erfindungsgemäßen Mittel kommen besonders dann zur Geltung, wenn diese so formuliert werden, daß sie einen pH-Wert in der Nähe des Neutralpunktes aufweisen. Mittel mit pH-Werten im Bereich von 5,5 - 7,5, insbesondere von 6,5 - 7,5, sind daher bevorzugt.

Die erfindungsgemäßen Mittel können in einer Vielzahl von Konsumentenprodukten wie Haarshampoos, Schaumbädern, Duschbädern, flüssigen Seifen und manuellen Geschirrspülmitteln Verwendung finden.

Diese Produkte enthalten neben den beschriebenen Tensidkombination die üblichen Bestandteile wie Emulgatoren, Ölkomponenten, Fette und Wachse, Lösungsvermittler, Verdickungsmittel, Überfettungsmittel, biogene Wirkstoffe, Filmbildner, Duftstoffe, Farbstoffe, Perlglanzmittel, Schaumstabilisatoren, Konservierungsmittel und pH-Regulatoren.

Als weitere Emulgatoren können die in kosmetischen Zubereitungen üblichen Substanzen wie z. B. Fettsäurepartialglyceride, Fettsäuresorbitanpartialester und deren Ethoxylate, Seifen, Fettalkoholpolyglykolether, Lanolin, Wollfettalkohole und Alkylphosphate verwendet werden.

Übliche Ölkomponenten sind Substanzen wie Paraffinöl, Pflanzenöle, Fettsäureester, Squalan und 2-Octyldodecanol, während als Fette und Wachse beispielsweise Walrat, Bienenwachs, Montanwachs, Paraffin und Cetyl-stearylalkohol Verwendung finden.

Als Lösungsvermittler werden üblicherweise niedrige ein- oder mehrwertige Alkohole wie Ethanol, Isopropanol, Ethylenglykol, Propylenglykol, Glycerin, 1,3-Butylenglykol und Diethylenglykol verwendet.

Als Überfettungsmittel können Substanzen wie polyethoxylierte Lanolinderivate, Lecithinderivate und Fettsäurealkanolamide verwendet werden, wobei die letzteren gleichzeitig auch als Schaumstabilisatoren dienen.

Geeignete Verdickungsmittel sind beispielsweise Polysaccharide, insbesondere Xanthan-Gum, Guar-Gum, Agar-Agar, Alginate und Tylosen, sowie Carboxymethylcellulose und Hydroxyethylcellulose, ferner höhermolekulare Polyethylenglykolmono- und -diester von Fettsäuren, Polyacrylate, Polyvinylalkohol und Polyvinylpyrrolidon und schließlich Elektrolyte wie Kochsalz und Ammoniumchlorid, gewünschtenfalls in Kombination mit Alkylethersulfaten.

Unter biogenen Wirkstoffen sind Pflanzenextrakte, Eiweißabbauprodukte und Vitaminkomplexe zu verstehen.

Gebräuchliche Filmbildner sind beispielsweise Polyvinylpyrrolidon, Vinylpyrrolidon-Vinylacetat-Copolymerisate, Polymere der Acrylsäurereihe, quaternäre Cellulose-Derivate und ähnliche Verbindungen.

Als Konservierungsmittel eignen sich die in der Anlage zur Kosmetikverordnung aufgeführten Substanzen.

Als Perlglanzmittel kommen insbesondere Glykoldistearinsäureester wie Ethylenglykoldistearat, aber auch Fettsäuremonoglykolester in Betracht.

Als Farbstoffe können die für kosmetischen Zwecke geeigneten und zugelassenen Substanzen verwendet werden, wie sie beispielsweise in der Publikation "Kosmetische Färbemittel" der Farbstoffkommission der Deutschen Forschungsgemeinschaft, veröffentlicht im Verlag Chemie, Weinheim, 1984, zusammengestellt sind. Diese Farbstoffe werden üblicherweise in Konzentrationen von 0,001 bis 0,1 Gew.-%, bezogen auf die gesamte Mischung, eingesetzt.

Weitere Komponenten der erfindungsgemäßen Mittel sind gewünschtenfalls Duftstoffe und Substanzen, die der Einstellung des pH-Wertes der Mittel dienen.

Die erfindungsgemäßen Mischungen werden bevorzugt in Produkten eingesetzt, die zum Waschen, Färben, Wellen oder Spülen von Haaren dienen. Insbesondere eignen sich die Mischungen für Shampoos zur Haarwäsche.

Die Erfindung wird durch die nachfolgenden Beispiele näher erläutert, ohne sie darauf zu beschränken.

Beispiele

1. Hautverträglichkeits-Untersuchungen

Zur Bestimmung der Hautverträglichkeit der Tensidmischungen wurde die von Zeidler und Reese entwickelte in-vitro-Methode verwendet, die in der Zeitschrift Ärztliche Kosmetologie 13, 39-45 (1983) ausführlich dargestellt ist.

Als Maß für die Hautverträglichkeit der Tensidmischungen diente die Quellung von Schweine-Epidermis. Dazu wurde die benötigte Epidermis unmittelbar nach der Schlachtung junger Schweine gewonnen und tiefgekühlt gelagert.

Für die Messung wurden ausgestanzte Epidermisstreifen der Größe 1 cm x 6 cm 30 Minuten lang in die Tensidlösungen getaucht, die jeweils 2 Gew.-% Aktivsubstanz enthielten, auf 39 °C temperiert und auf pH = 6,5 eingestellt waren. Sodann wurde nach kurzem Spülen und Entfernen des anhaftenden Wasser durch leichtes Abpressen unter definierten Bedingungen das Gewicht der gequollenen Streifen bestimmt. Anschließend wurden die Streifen 24 Stunden über Calciumchlorid entwässert und erneut gewogen. Um Einflüsse auszuschalten, die auf spezifische Eigenschaften des jeweiligen Tieres oder den Entnahmeort (Rücken, Seite) zurückgehen, wurde jeweils eine Standardmessung durchgeführt. Dabei wird ein unmittelbar benachbarter Epidermisstreifen in gleicher Weise mit Wasser anstelle der Tensidlösung behandelt.

Die Meßwerte t für die Tensid-Behandlung und w für die Behandlung mit Wasser ergeben sich aus der Beziehung:

$$t, w = \frac{\text{Gewicht (gequollene Epidermis)} - \text{Gewicht (trockene Epidermis)}}{\text{Gewicht (trockene Epidermis)}}$$

Die standardisierte, relative Quellungsänderung Q ist schließlich definiert als

$$Q = \left(\frac{t}{w} - 1\right) * 100\%$$

Der Q-Wert der wasserbehandelten Haut ist somit definitionsgemäß 0 %; negative Werte weisen auf quellungshemmende Eigenschaften hin.

In den Beispielen wurden die im folgenden aufgeführten Tensidkomponenten verwendet. Die Zahl vor "EO" gibt jeweils den mittleren Ethoxylierungsgrad (x in Formel I und II) an.

A1: Hydroxy-oleyl-polyoxyethyl(3 EO)-sulfonat
Die Substanz wurde analog zu der in den Beispielen 2.1 und 2.2 der Deutschen Patentanmeldung 37 25 030.2 offenbarten Vorschrift hergestellt. Als Ausgangsverbindung diente ein technischer Oleylalkohol, der unter der Bezeichnung "HD-Ocenol(R) 90/95" von der Anmelderin vertrieben wird und durch eine Jodzahl von 94 und eine Hydroxylzahl von 210 gekennzeichnet ist.

A2: Hydroxy-oleyl-polyoxyethyl(5 EO)-sulfonat
Die Substanz wurde nach der in den Beispielen 3.1 und 3.2 der Deutschen Patentanmeldung 37 25 030.2 offenbarten Herstellvorschrift synthetisiert. Als Ausgangsverbindung diente ebenfalls der technische Oleylalkohol "HD-Ocenol(R) 90/95".

A3: Hydroxy-oleyl-polyoxyethyl(10 EO)-sulfonat
Die Substanz ist wurde nach der in den Beispielen 4.1 und 4.2 der Deutschen Patentanmeldung 37 25 030.2 offenbarten Herstellvorschrift synthetisiert. Als Ausgangsverbindung diente ebenfalls der technische Oleylalkohol "HD-ocenol(R) 90/95".

B1a: Tensidmischung BES1
Die Tensidmischung BES1 wurde wie folgt hergestellt:
Ein Gemisch aus 96 kg eines Adduktes von 10 Mol Ethylenoxid an einem Kokosfettalkohol $C_{12}$-$C_{18}$ (54 Gewichtsprozent $C_{12}$, 22 Gewichtsprozent $C_{14}$, 10 Gewichtsprozent $C_{16}$, 12 Gewichtsprozent $C_{18}$), 113 kg eines Adduktes von 2 Mol Ethylenoxid an einem Kokosfettalkohol $C_{12}$-$C_{14}$ (73 Gewichtsprozent $C_{12}$, 27 Gewichtsprozent $C_{14}$) und 24,5 kg eines Adduktes von 2 Mol Ethylenoxid an einem Oleyl-Cetyl-Alkohol (65 Gewichtsprozent $C_{18}$, 30 Gewichtsprozent $C_{16}$, 5 Gewichtsprozent $C_{14}$, Jodzahl 50) wurde mit 74,4 kg Chlorsulfonsäure bei einer Temperatur von 10 bis 20 °C in einem kontinuierlichen Reaktor zum Schwefelsäurehalbester umgesetzt und anschließend mit 55,5 kg einer 50 %igen wässrigen Natriumhydroxidlösung, verdünnt mit 660 kg Wasser, neutralisiert. Das dabei erhaltene Alkylethersulfat hatte einen Aniontensid-Gehalt von 0,592 Mol/kg (bestimmt durch Zweiphasentitration nach DGF-Einheitsmethode H-III-10).
Zu 880 g dieses Ethersulfats wurden 28,2 g $MgSO_4 \cdot 7\ H_2O$ (0,114 Mol), 4,6 g Trinatriumcitrat und 65 g Natriumchlorid (1,1 Mol) gegeben. Die so erhaltene Tensidmischung BES1 hatte einen Aniontensid-Gehalt von ca. 0,59 Mol/kg entsprechend ca. 28 Gewichtsprozent (berechnet mit einem mittleren Molekulargewicht der Aniontenside von 475).

B2a: Texapon(R) 1296 (HENKEL)
Natriumlaurylsulfat
B3a: Akypo(R) RLM 100 NV (CHEM-Y)
Wäßrige Lösung von $C_{12-14}$-Alkyl-O-($CH_2$-$CH_2$-O)$_{10}$-$CH_2$-COONa (22% Aktivsubstanz)
B4a: Texapon(R) SB 3 (HENKEL)
Sulfobernsteinsäurehalbester auf Basis eines $C_{12-14}$-Alkylpolyglykol(3 EO)ethers, Dinatriumsalz (40 % Aktivsubstanz); CTFA-Bezeichnung Disodium Laurethsulfosuccinate

Die Ergebnisse der Quellungsmessungen sind in der folgenden Tabelle zusammengestellt. Die Mengen-Angabe bezieht sich dabei auf die Gesamtmenge von 2 % Aniontensid.

| Tensid-Mischung | Tensid A | Menge | Tensid B | Menge | Q-Wert |
|---|---|---|---|---|---|
| - | A1 | 100 % | - | - | 14 ± 6 |
| - | A2 | 100 % | - | - | 28 ±13 |
| - | A3 | 100 % | - | - | 20 ± 6 |
| - | - | - | B1a | 100 % | 36 ±11 |
| - | - | - | B2a | 100 % | 173 ±15 |
| - | - | - | B3a | 100 % | 18 ± 6 |
| - | - | - | B4a | 100 % | 32 ±10 |
| M1 | A2 | 40 % | B1a | 60 % | 33 ± 7 |
| M2 | A2 | 60 % | B1a | 40 % | 20 ± 7 |
| M3 | A3 | 20 % | B1a | 80 % | 13 ± 7 |
| M4 | A3 | 40 % | B1a | 60 % | 3 ± 7 |
| M5 | A3 | 60 % | B1a | 40 % | - 11 ± 5 |
| M6 | A3 | 20 % | B2a | 80 % | 79 ±16 |
| M7 | A3 | 20 % | B3a | 80 % | 13 ± 5 |
| M8 | A3 | 80 % | B3a | 20 % | 11 ± 5 |
| M9 | A1 | 60 % | B4a | 40 % | 12 ± 5 |
| M10 | A2 | 20 % | B4a | 80 % | 19 ± 8 |
| M11 | A2 | 60 % | B4a | 40 % | 17 ± 9 |
| M12 | A3 | 40 % | B4a | 60 % | 14 ± 9 |
| M13 | A3 | 80 % | B4a | 20 % | - 10 ± 8 |

2. Bestimmung des Schaumverhaltens

Das Schaumverhalten der Tenside und Tensidmischungen wurde mit einer motorisierten Schlag-Schaum-Apparatur in Anlehnung an DIN 53902 bestimmt.

Zur Bestimmung wurden 340 ml Tensidlösung (2 Gew.-% Aktivsubstanz in Leitungswasser aus Düsseldorf-Holthausen mit 18 °dH) hergestellt. Der Schaum wurde bei Raumtemperatur mit einer Lochplatte (Bohrungen von 1 mm Durchmesser, 10 Schläge bei einer Frequenz von 50 Schlägen/min, 13 cm Hub) erzeugt; er war sehr feinporig und entsprach somit weitgehend einem beim Shampoonieren auf dem Kopf entstehenden Schaum. Die Messungen wurden ohne Fettbelastung der Tensidlösung als Doppelbestimmung durchgeführt.

Als Vergleich wurde das Schaumverhalten einer als gut schäumend bekannten Mischung aus
42 Gewichtsprozent Texapon(R) N 25[1], (= 12 % Aktivsubstanz)
10 Gewichtsprozent Dehyton(R) K[2], (= 3 % Aktivsubstanz)
1 Gewichtsprozent Comperlan(R) LS[3] (= 1 % Aktivsubstanz) und
47 Gewichtsprozent Wasser
bestimmt.

[1] Wäßrige Lösung von Natriumlaurylethersulfat (ca. 28 % Aktivsubstanz) (HENKEL)

[2] Wäßrige Lösung eines Fettsäureamid-Derivat mit Betainstruktur der Formel R-CONH-$(CH_2)_3$-$N^+(CH_3)_2$-$CH_2$-COO- mit der CTFA-Bezeichnung Cocamidopropyl Betaine (ca. 30 % Aktivsubstanz, ca. 5 % NaCl, (HENKEL)

[3] Mischung von Kokosfettsäurediethanolamid (ca. 70 %) und Laurylalkohol + 2 Ethylenoxid (ca. 20 %); Rest: Wasser, freies Amin, freie Fettsäure, Ester (HENKEL)

Diese Mischung wurde mit Wasser verdünnt und ebenfalls in Form einer Lösung mit 2 Gew.-% Aktivsubstanz eingesetzt. Es wurden folgende Schaummengen gemessen:
nach 1 Minute: 240 ml
nach 3 Minuten: 210 ml
nach 5 Minuten: 190 ml

Die gemessenen Schaummengen der erfindungsgemäßen Tensidlösungen sind in der folgenden Tabelle in Prozent bezogen auf die Schaummenge der Vergleichssubstanz angegeben.

| Tensid/ Tensid-Mischung | relative Schaummenge nach 1 min | nach 3 min | nach 5 min |
|---|---|---|---|
| A1 | 71 | 33 | 21 |
| A2 | 54 | 33 | 21 |
| A3 | 74 | 70 | 72 |
| B1a | 87 | 95 | 95 |
| B2a | 65 | 65 | 72 |
| B3a | 70 | 75 | 78 |
| B4a | 70 | 67 | 72 |
| M1 | 79 | 86 | 89 |
| M2 | 83 | 81 | 84 |
| M3 | 87 | 90 | 89 |
| M4 | 87 | 85 | 83 |
| M5 | 78 | 80 | 83 |
| M6 | 78 | 80 | 83 |
| M7 | 65 | 70 | 78 |
| M8 | 61 | 60 | 67 |
| M9 | 70 | 67 | 72 |
| M10 | 87 | 86 | 94 |
| M11 | 70 | 57 | 39 |
| M12 | 91 | 81 | 89 |
| M13 | 56 | 52 | 50 |

3. Hautverträglichkeit von Mischungen mit ampholytischen/zwitterionischen Tensiden

Es wurden wäßrige Systeme untersucht, die 1,6 Gew.-% an Aktivsubstanz der Tensid-Mischung M5 und 0,4 Gew.-% an Aktivsubstanz des ampholytischen/zwitterionischen Tensids Z enthielten. In der Tabelle ist auch der Wert für eine Mischung, die 2 Gew.-% an Aktivsubstanz der binären Tensid-Mischung M5 enthält, angegeben.

Es wurden folgende Q-Werte gemessen:

| Tensid Z | Q-Wert |
|---|---|
| - | - 11 ± 5 |
| Dehyton(R) AB 30[4] | - 6 ± 8 |
| Dehyton(R) CB[5] | 12 ± 5 |
| Dehyton(R) G[6] | 7 ±10 |
| Dehyton(R) G-SF[7] | - 14 ± 6 |
| Dehyton(R) K | - 7 ± 8 |

[4] Wäßrige Lösung eines Fettamin-Derivates mit Betainstruktur, CTFA-Bezeichnung: Coco-Betaine (ca. 30 % Aktivsubstanz, ca. 6 % NaCl) (HENKEL)

[5] Wäßrige Lösung eines Fettamin-Derivates mit Betainstruktur, CTFA-Bezeichnung: Coco-Betaine (ca. 31 % Aktivsubstanz, ca. 6,5 % NaCl) (HENKEL)

[6] Wäßrige Lösung von N-Hydroxyethyl-N-Kokoalkylamidoethyl-glycinat-Natriumsalz, CTFA-Bezeichnung Cocoamphodiacetate, (ca. 30 % Aktivsubstanz, ca. 7 % NaCl) (HENKEL)

[7] Wäßrige Lösung von N-Hydroxyethyl-N-Alkylamidoethyl-propionat, CTFA-Bezeichnung Cocoamphodipropionate, (ca. 40 % Aktivsubstanz) (HENKEL)

Das Schaumverhalten dieser ternären Tensidmischungen wurde in gleicher Weise wie bei den binären Tensid-Mischungen bestimmt. Die wäßrigen Mischungen enthielten 1,6 Gew.-% an Aktivsubstanz der binären Tensid-Mischung M5 und 0,4 Gew.- % an Aktivsubstanz der dritten Tensidkomponente Z.

Die gemessenen Schaummengen der erfindungsgemäßen Tensidlösungen sind in der folgenden Tabelle in Prozent bezogen auf die Schaummenge der Vergleichssubstanz angegeben. In der Tabelle ist auch der Wert für eine Mischung, die 2 Gew.-% an Aktivsubstanz der binären Tensid-Mischung M5 enthält, angegeben.

| Tensidkomponente Z | relative Schaummenge | | |
|---|---|---|---|
| | nach 1 min | nach 3 min | nach 5 min |
| - | 78 | 80 | 83 |
| Dehyton(R) K | 87 | 90 | 100 |
| Dehyton(R) AB 30 | 96 | 95 | 100 |
| Dehyton(R) G | 100 | 90 | 100 |
| Dehyton(R) G-SF | 100 | 95 | 105 |
| Dehyton(R) CB | 100 | 100 | 105 |

4. Anwendungsbeispiele:

1. klares Shampoo, speziell geeignet für fettige Haare

| Komponente | Gewichtsteile |
|---|---|
| Tensid A3 | 9,6 |
| Tensidmischung B1a | 8,6 |
| Dehyton(R) K | 10,0 |
| Dehydol(R) LS 3[8] | 2,0 |
| Polymer JR(R) 400[9] | 0,1 |
| Wasser, Farbstoffe, Parfüm, Konservierungsmittel | ad 100 |

[8] $C_{12}$-$C_{14}$-Fettalkohol + 3 Ethylenoxid (HENKEL)

[9] Hydroxyethylcellulose, quaterniert (UNION-CARBIDE)

Die Haare wiesen nach der Behandlung mit diesem Shampoo eine sehr gute Naßkämmbarkeit und eine sehr gute Trockenkämmbarkeit auf

2. Klares Shampoo, speziell geeignet für strapazierte Haare

| Komponente | Gewichtsteile |
|---|---|
| Tensid A3 | 9,6 |
| Tensid B4a | 6,0 |
| Dehyton(R) K | 10,0 |
| Dehydol(R) LS 3 | 4,0 |
| Polymer JR(R) 400 | 0,1 |
| Wasser,Farbstoffe, Parfüm, Konservierungsmittel | ad 100 |

Die Haare wiesen nach der Behandlung mit diesem Shampoo eine sehr gute Naßkämmbarkeit und eine sehr gute Trockenkämmbarkeit auf.

3. Cremiges, klares Shampoo

| Komponente | Gewichtsteile |
|---|---|
| Tensid A3 | 6,0 |
| Tensid B4a | 15,0 |
| Dehydol(R) LS 3 | 4,0 |
| Aminoxid WS(R) 35[10] | 8,6 |
| Polymer JR(R) 400 | 0,1 |
| Glucamate DOE(R) 120[11] | 5,0 |
| Wasser, Farbstoffe, Parfüm, Konservierungsmittel | ad 100 |

[10] Wäßrige Lösung von Dimethylkokosacylamidopropylaminoxid (ca. 35 % Aktivsubstanz) (GOLDSCHMIDT)

[11] PEG-120-Methylglucosedioleat (AMERCHOL)

Die Haare wiesen nach der Behandlung mit diesem Shampoo eine ausgezeichnete Naßkämmbarkeit und eine ausgezeichnete Trockenkämmbarkeit auf.

4. Besonders mildes Shampoo mit feinem cremigem Schaum

| Komponente | Gewichtsteile |
|---|---|
| Tensid A3 | 3,0 % |
| Texapon (R) N 25 | 42,0 % |
| Dehydol (R) LS 3 | 2,0 % |
| Alkylglucosid APG-600[12] | 4,0 % |
| Polymer JR (R) 400 | 0,1 % |
| NaCl | 1,0 % |
| Wasser, Konservierungsmittel, Parfüm | ad 100 |

[12] Wäßrige Lösung von $RO(Z)_x$ mit Z = Glucose, x = 1,4 und R = n-Alkyl ($C_{12-14}$), (50 % Aktivsubstanz) (HORIZON)

## Patentansprüche

1. Oberflächenaktive wäßrige Mischungen enthaltend mindestens zwei unterschiedliche Aniontenside, dadurch gekennzeichnet, daß als Aniontenside
   a) ein Gemisch oberflächenaktiver innenständiger Ethersulfonate (A), das ganz oder überwiegend aus Verbindungen der Formeln (I) und/oder (II)

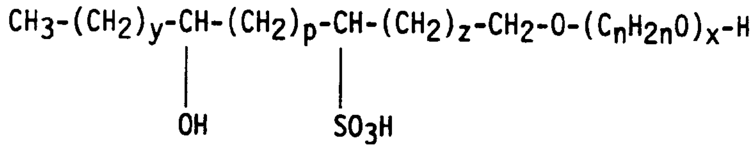

(I)

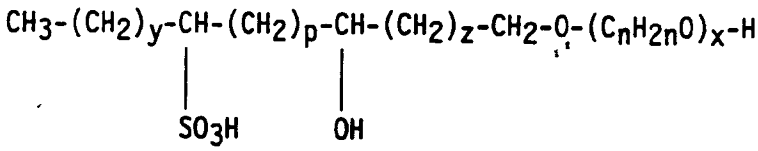

(II)

   oder deren Alkali-, Erdalkali-, Aluminium- und Ammoniumsalzen zusammengesetzt ist, wobei y und z = 0 oder Zahlen von 1 bis 18, p = 0, 1 oder 2 und die Summe (y+z+p) eine Zahl von 12 bis 18, x = 0 oder eine ganze Zahl bis 30 und n eine ganze Zahl von 2 bis 4 sein kann,
   und
   b) mindestens eine weitere Verbindung (B), ausgewählt aus der Gruppe der Fettalkoholethersulfate, Fettalkoholsulfate, Ethercarbonsäuren, Sulfobernsteinsäureester, Acylsarkoside, Acyltauride, Acylisethionate, α-Olefin-sulfonate und sekundären Alkylsulfonate,
   enthalten sind.

2. Mischungen nach Anspruch 1, dadurch gekennzeichnet, daß bei den Verbindungen (A) gemäß Formel (I) oder (II) n = 2 und x eine ganze Zahl von 3 bis 15, insbesondere von 5 bis 10 ist.

3. Mischungen nach einem der Ansprüche 1 oder 2, dadurch gekennzeichnet, daß sie als Komponente (A) innenständige Ethersulfonate mit (y+z+p) = 14 und x = 10 enthalten.

4. Mischungen nach einem der Ansprüche 1 bis 3, dadurch gekennzeichnet, daß sie Verbindungen (A) gemäß Formel (I) oder (II) in Mengen von 1 bis 99 Gew.-%, bezogen auf die Gesamtmenge der Aniontenside, enthalten.

5. Mischungen nach einem der Ansprüche 1 bis 4, dadurch gekennzeichnet, daß sie als Aniontensid (B1) Fettalkoholethersulfate gemäß Formel (III) enthalten,
   $$R\text{-}O\text{-}(C_mH_{2m}O)_v\text{-}SO_3X \qquad (III)$$
   in der R ein gesättigter oder olefinisch ungesättigter, linearer oder verzweigter Alkylrest mit 8 bis 22 C-Atomen, m 2 oder 3, v eine Zahl von 1 bis 15 und X Wasserstoff, ein Alkalimetall, ein Erdalkalimetall, Aluminium, eine Ammoniumgruppe oder eine Alkyl- oder Alkylolammoniumgruppe mit 1 bis 4 C-Atomen in jeder Alkyl- oder Alkylolgruppe ist.

6. Mischungen nach einem der Ansprüche 1 bis 5, dadurch gekennzeichnet, daß sie die Aniontenside (A) und (B1) in einem Mengenverhältnis von etwa 10:90 bis etwa 70:30, insbesondere von 20:80 bis 60:40, enthalten.

7. Mischungen nach einem der Ansprüche 1 bis 6, dadurch gekennzeichnet, daß sie als Aniontensid (B2) Fettalkoholsulfate gemäß Formel (IV) enthalten,
   $$R\text{-}O\text{-}SO_3X \qquad (IV)$$
   in der R ein gesättigter oder olefinisch ungesättigter, linearer oder verzweigter Alkylrest mit 8 bis 22 C-Atomen und X Wasserstoff, ein Alkalimetall, ein Erdalkalimetall, Aluminium, eine Ammoniumgruppe oder eine Alkyl- oder Alkylolammoniumgruppe mit 1 bis 4 C-Atomen in jeder Alkyl- oder Alkylolgruppe ist.

8. Mischungen nach einem der Ansprüche 1 bis 7, dadurch gekennzeichnet, daß sie die Aniontenside (A)

und (B2) in einem Mengenverhältnis von etwa 10:90 bis etwa 50:50, insbesondere von 10:90 bis 30:70, enthalten.

**9.** Mischungen nach einem der Ansprüche 1 bis 8, dadurch gekennzeichnet, daß sie als Aniontensid (B3) Ethercarbonsäuren gemäß Formel (V) enthalten,

$$R\text{-}O\text{-}(C_mH_{2m}O)_v\text{-}CH_2\text{-}COOX \qquad (V)$$

in der R ein gesättigter oder olefinisch ungesättigter, linearer oder verzweigter Alkylrest mit 8 bis 22 C-Atomen, m 2 oder 3, v eine Zahl von 1 bis 15 und X Wasserstoff, ein Alkalimetall, ein Erdalkalimetall, Aluminium, eine Ammoniumgruppe oder eine Alkyl- oder Alkylolammoniumgruppe mit 1 bis 4 C-Atomen in jeder Alkylolgruppe ist.

**10.** Mischungen nach einem der Ansprüche 1 bis 9, dadurch gekennzeichnet, daß sie die Aniontenside (A) und (B3) in einem Mengenverhältnis von etwa 10:90 bis etwa 90:10, insbesondere von 20:80 bis 80:20, enthalten.

**11.** Mischungen nach einem der Ansprüche 1 bis 10, dadurch gekennzeichnet, daß sie als Aniontensid (B4) Sulfobernsteinsäureester gemäß Formel (VI) enthalten,

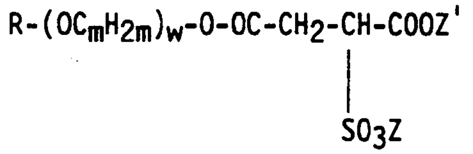

(VI)

in der R ein gesättigter oder olefinisch ungesättigter, linearer oder verzweigter Alkylrest mit 8 bis 22 C-Atomen, m 2 oder 3, w = 0 oder eine Zahl von 1 bis 15 und Z ein Alkali- metall, ein Erdalkalimetall, Aluminium, eine Ammoniumgruppe, eine Alkyl- oder Alkylolammoniumgruppe mit 1 bis 4 C-Atomen in jeder Alkyl- oder Alkylolgruppe und Z' ein Alkalimetall, ein Erdalkalimetall, Aluminium, eine Ammoniumgruppe, eine Alkyl- oder Alkylolammoniumgruppe mit 1 bis 4 C-Atomen in jeder Alkyl- oder Alkylolgruppe oder eine Gruppe R ist.

**12.** Mischungen nach einem der Ansprüche 1 bis 11, dadurch gekennzeichnet, daß sie die Aniontenside (A) und (B4) in einem Mengenverhältnis von etwa 10:90 bis etwa 90:10, insbesondere von 20:80 bis 80:20, enthalten.

**13.** Mischungen nach einem der Ansprüche 1 bis 12, dadurch gekennzeichnet, daß sie zusätzlich 0,5-20 Gew.-%, insbesondere 1-10 Gew.-%, an ampholytischen und/oder zwitterionischen Tensiden, jeweils bezogen auf die gesamte Mischung, enthalten.

**14.** Mischungen nach einem der Ansprüche 1 bis 13, dadurch gekennzeichnet, daß sie einen pH-Wert von 5,5-7,5, insbesondere von 6,5-7,5 aufweisen.

**15.** Mischungen nach einem der Ansprüche 1 bis 14, dadurch gekennzeichnet, daß sie als Shampoo formuliert sind.

**16.** Verwendung von Mischungen nach einem der Ansprüche 1 bis 15 zur Haarbehandlung.

## Claims

**1.** Surface-active aqueous mixtures containing at least two different anionic surfactants, characterized in that they contain as anionic surfactants

a) a mixture of surface-active internal ether sulfonates (A), which consists completely or predominantly of compounds corresponding to formulae (I) and/or (II)

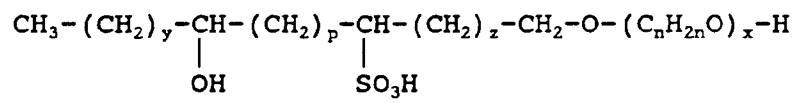

(I)

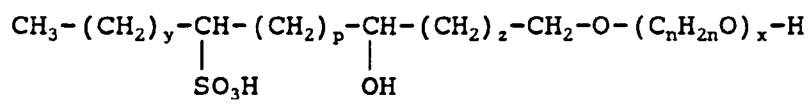

(II)

in which y and z = 0 or numbers of 1 to 18, p = 0, 1 or 2 and the sum (y+z+p) is a number of 12 to 18, x = 0 or an integer of up to 30 and n may be an integer of 2 to 4,
or alkali metal, alkaline earth metal, aluminium and ammonium salts thereof,
and
b) at least one other compound (B) selected from the group consisting of fatty alcohol ether sulfates, fatty alcohol sulfates, ether carboxylic acids, sulfosuccinic acid esters, acyl sarcosides, acyl taurides, acyl isethionates, $\alpha$-olefin sulfonates and secondary alkyl sulfonates.

**2.** Mixtures as claimed in claim 1, characterized in that, in the compounds (A) corresponding to formula (I) or (II), n = 2 and x is an integer of 3 to 15 and, more particularly, 5 to 10.

**3.** Mixtures as claimed in claim 1 or 2, characterized in that they contain internal ether sulfonates with (y+z+p) = 14 and x = 10 as component (A).

**4.** Mixtures as claimed in any of claims 1 to 3, characterized in that they contain compounds (A) corresponding to formula (I) or (II) in quantities of 1 to 99% by weight, based on the total quantity of anionic surfactants.

**5.** Mixtures as claimed in any of claims 1 to 4, characterized in that they contain as the anionic surfactant (B1) fatty alcohol ether sulfates corresponding to formula (III)

$$R\text{-}O\text{-}(C_mH_{2m}O)_v\text{-}SO_3X \qquad (III)$$

in which R is a saturated or olefinically unsaturated, linear or branched $C_{8-22}$ alkyl radical, m = 2 or 3, v is a number of 1 to 15 and X is hydrogen, an alkali metal, an alkaline earth metal, aluminium, an ammonium group or an alkyl or alkylol ammonium group containing 1 to 4 carbon atoms in the each alkyl or alkylol group.

**6.** Mixtures as claimed in any of claims 1 to 5, characterized in that they contain the anionic surfactants (A) and (B1) in a quantitative ratio of from about 10:90 to about 70:30 and, more particularly, from 20:80 to 60:40.

**7.** Mixtures as claimed in any of claims 1 to 6, characterized in that they contain as the anionic surfactant (B2) fatty alcohol sulfates corresponding to formula (IV)

$$R\text{-}O\text{-}SO_3X \qquad (IV)$$

in which R is a saturated or olefinically unsaturated, linear or branched $C_{8-22}$ alkyl radical and X is hydrogen, an alkali metal, an alkaline earth metal, aluminium, an ammonium group or an alkyl or alkylol ammonium group containing 1 to 4 carbon atoms in each alkyl or alkylol group.

**8.** Mixtures as claimed in any of claims 1 to 7, characterized in that they contain the anionic surfactants (A) and (B2) in a quantitative ratio of from about 10:90 to about 50:50 and, more particularly, from 10:90 to 30:70.

**9.** Mixtures as claimed in any of claims 1 to 8, characterized in that they contain as the anionic surfactant (B3) ether carboxylic acids corresponding to formula (V)

$$R\text{-}O\text{-}(C_mH_{2m}O)_v\text{-}CH_2\text{-}COOX \qquad (V)$$

in which R is a saturated or olefinically unsaturated, linear or branched $C_{8-22}$ alkyl radical, m = 2 or 3, v is a number of 1 to 15 and X is hydrogen, an alkali metal, an alkaline earth metal, aluminium, an ammonium group or an alkyl or alkylol ammonium group containing 1 to 4 carbon atoms in each alkyl or alkylol group.

10. Mixtures as claimed in any of claims 1 to 9, characterized in that they contain the anionic surfactants (A) and (B3) in a quantitative ratio of from about 10:90 to about 90:10 and, more particularly, from 20:80 to 80:20.

11. Mixtures as claimed in any of claims 1 to 10, characterized in that they contain as the anionic surfactant (B4) sulfosuccinic acid esters corresponding to formula (VI)

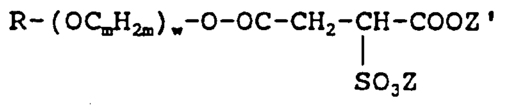

(VI)

in which R is a saturated or olefinically unsaturated, linear or branched $C_{8-22}$ alkyl radical, m = 2 or 3, w = 0 or is a number of 1 to 15 and Z is an alkali metal, an alkaline earth metal, aluminium, an ammonium group, an alkyl or alkylol ammonium group containing 1 to 4 carbon atoms in each alkyl or alkylol group and Z' is an alkali metal, an alkaline earth metal, aluminium, an ammonium group, an alkyl or alkylol ammonium group containing 1 to 4 carbon atoms in each alkyl or alkylol group or is a group R.

12. Mixtures as claimed in any of claims 1 to 11, characterized in that they contain the anionic surfactants (A) and (B4) in a quantitative ratio of from about 10:90 to about 90:10 and, more particularly, from 20:80 to 80:20.

13. Mixtures as claimed in any of claims 1 to 12, characterized in that they additionally contain 0.5 to 20% by weight and more particularly 1 to 10% by weight ampholytic and/or zwitterionic surfactants, based on the mixture as a whole.

14. Mixtures as claimed in any of claims 1 to 13, characterized in that they have a pH value of 5.5 to 7.5 and, more particularly, 6.5 to 7.5.

15. Mixtures as claimed in any of claims 1 to 14, characterized in that they are formulated as a shampoo.

16. The use of the mixtures claimed in any of claims 1 to 15 for the treatment of hair.

## Revendications

1. Mélanges aqueux de tensioactifs, renfermant au moins deux surfactifs anioniques différents, caractérisés en ce qu'ils renferment comme tensioactifs anioniques
   a) un mélange d'éthersulfonates tensioactifs internes (A), qui se compose en totalité ou en majeure partie de composés des formules (I) et/ou (II)

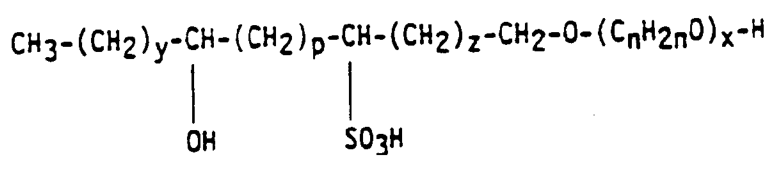

(I)

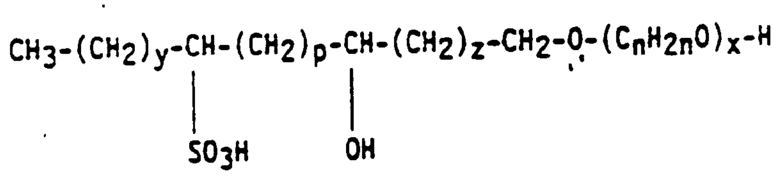

(II)

ou de leurs sels de métaux alcalins, de métaux alcalino-terreux, d'aluminium et d'ammonium, formules dans lesquelles y et z sont égaux à 0 ou à des nombres allant de 1 à 18, p est égal à 0, à 1 ou à 2 et la somme (y+z+p) équivaut à un nombre allant de 12 à 18, x est égal à 0 ou à un nombre entier allant jusqu'à 30 et n peut être un nombre entier allant de 2 à 4,

et

b) au moins un autre composé (B) sélectionné dans le groupe des éthersulfates d'alcools gras, sulfates d'alcools gras, acides éthercarboxyliques, esters d'acide sulfosuccinique , acylsarcosides, acyltaurides, acyliséthionates, alphaoléfinesulfonates et alkylsulfonates secondaires.

**2.** Mélanges selon la revendication 1, caractérisés en ce que dans les composés (A) conformes à la formule (I) ou (II), n est égal à 2 et x équivaut à un nombre entier allant de 3 à 15, en particulier de 5 à 10.

**3.** Mélanges selon l'une des revendications 1 ou 2, caractérisés en ce qu'ils renferment comme composant (A), des éthersulfonates internes avec (y+z+p) = 14 et x = 10.

**4.** Mélanges selon l'une des revendications 1 à 3, caractérisés en ce qu'ils renferment des composés (A) conformes à la formule (I) ou (II), en proportions allant de 1 à 99 % en poids, par rapport à la quantité totale des tensioactifs anioniques.

**5.** Mélanges selon l'une des revendications 1 à 4, caractérisés en ce qu'ils renferment comme tensioactif anionique (B1) des éthersulfates d'alcools gras conformes à la formule (III),

$$R\text{-}O\text{-}(C_mH_{2m}O)_v\text{-}SO_3X \qquad (III)$$

dans laquelle R représente un radical alkyle linéaire ou ramifié, saturé ou à insaturation oléfinique, comportant 8 à 22 atomes de C, m est égal à 2 ou à 3, v correspond à un nombre allant de 1 à 15 et X est l'hydrogène, un métal alcalin, un métal alcalino-terreux, l'aluminium, un groupe ammonium ou un groupe alkyl- ou alkylolammonium comportant 1 à 4 atomes de C dans chaque groupe alkyle ou alkylol.

**6.** Mélanges selon l'une des revendications 1 à 5, caractérisés en ce qu'ils renferment les tensioactifs anioniques (A) et (B1) dans un rapport de quantités compris entre environ 10:90 et environ 70:30, en particulier entre 20:80 et 60:40.

**7.** Mélanges selon l'une des revendications 1 à 6, caractérisés en ce qu'ils renferment comme tensioactif anionique (B2), des sulfates d'alcools gras conformes à la formule (IV),

$$R\text{-}O\text{-}SO_3X \qquad (IV)$$

dans laquelle R représente un radical alkyle linéaire ou ramifié, saturé ou à insaturation oléfinique, comportant 8 à 22 atomes de C et X est l'hydrogène, un métal alcalin, un métal alcalino-terreux, l'aluminium, un groupe ammonium ou un groupe alkyl- ou alkylolammonium comportant 1 à 4 atomes de C dans chaque groupe alkyle ou alkylol.

**8.** Mélanges selon l'une des revendications 1 à 7, caractérisés en ce qu'ils renferment les tensioactifs anioniques (A) et (B2) dans un rapport de quantités compris entre environ 10:90 et environ 50:50, en particulier entre 10:90 et 30:70.

**9.** Mélanges selon l'une des revendications 1 à 8, caractérisés en ce qu'ils renferment comme tensioactif anionique (B3), des acides éthercarboxyliques conformes à la formule (V),

$$R\text{-}O\text{-}(C_mH_{2m}O)_v\text{-}CH_2\text{-}COOX \qquad (V)$$

dans laquelle R représente un radical alkyle linéaire ou ramifié, saturé ou à insaturation oléfinique, comportant 8 à 22 atomes de C, m est égal à 2 ou à 3, v correspond à un nombre allant de 1 à 15 et X est l'hydrogène, un métal alcalin, un métal alcalino-terreux, l'aluminium, un groupe ammonium ou un groupe alkyl- ou alkylolammonium comportant 1 à 4 atomes de C dans chaque groupe alkylol.

**10.** Mélanges selon l'une des revendications 1 à 9, caractérisés en ce qu'ils renferment les tensioactifs anioniques (A) et (B3) dans un rapport de quantités compris entre environ 10:90 et environ 90:10, en particulier entre 20:80 et 80:20.

**11.** Mélanges selon l'une des revendications 1 à 10, caractérisés en ce qu'ils renferment comme tensioactif anionique (B4) des esters d'acide sulfosuccinique conformes à la formule (VI),

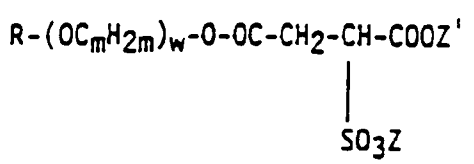

(VI)

dans laquelle R représente un radical alkyle linéaire ou ramifié, saturé ou à insaturation oléfinique, comportant 8 à 22 atomes de C, m est égal à 2 ou à 3, w correspond à 0 ou à un nombre allant de 1 à 15 et Z est un métal alcalin, un métal alcalino-terreux, l'aluminium, un groupe ammonium ou un groupe alkyle ou alkylolammonium comportant 1 à 4 atomes de C dans chaque groupe alkyle ou alkylol et Z' représente un métal alcalin, un métal alcalino-terreux, l'aluminium, un groupe ammonium ou un groupe alkyl- ou alkylolammonium comportant 1 à 4 atomes de C dans chaque groupe alkyle ou alkylol ou un groupe R.

**12.** Mélanges selon l'une des revendications 1 à 11, caractérisés en ce qu'ils renferment les tensioactifs anioniques (A) et (B4) dans un rapport de quantités compris entre environ 10:90 et environ 90:10, en particulier entre 20:80 et 80:20.

**13.** Mélanges selon l'une des revendications 1 à 12, caractérisés en ce qu'ils renferment en plus 0,5 à 20 % en poids, en particulier 1 à 10 % en poids de tensioactifs ampholytes et/ou zwitterioniques, chacun par rapport à la totalité du mélange.

**14.** Mélanges selon l'une des revendications 1 à 13, caractérisés en ce qu'ils présentent un pH de 5,5 à 7,5, en particulier de 6,5 à 7,5.

**15.** Mélanges selon l'une des revendications 1 à 14, caractérisés en ce qu'ils sont formulés comme shampooing.

**16.** Utilisation des mélanges selon l'une des revendications 1 à 15 pour le traitement des cheveux.